# EUROPÄISCHE PATENTANMELDUNG

(11) **EP 2 731 036 A2**
(43) Veröffentlichungstag der Anmeldung: **14.05.2014**
(21) Anmeldenummer: 13191919.3
(22) Anmeldetag: 07.11.2013
(51) Int. Cl.: G06F 19/00

(54) **Verfahren zur Erstellung virtueller Kurvendiagramme**

(30) Priorität: 09.11.2012 DE 102012110776
(71) Anmelder: Optiplan Gesellschaft für optische Planungsgeräte mit beschränkter Haftung, 40489 Düsseldorf (DE)
(72) Erfinder: Lerner, Sabine, 40489 Düsseldorf (DE); Wagner, Kai, 40489 Düsseldorf (DE)
(74) Vertreter: Stenger, Watzke & Ring

(57) **Zusammenfassung**

Die Erfindung betrifft ein Verfahren zur Erstellung virtueller Kurvendiagramme, welche patientenbezogene Daten aufweisen, durch einen Benutzer eines Computers. Um beispielsweise in Krankenhäusern moderne Computertechnologie nutzen zu können, ohne gleichzeitig aber einen fehlerträchtigen Umgewöhnungsprozess für die Benutzer hervorzurufen, schlägt die Erfindung vor, dass der Benutzer mittels einer Eingabeeinheit des Computers einen Bereich eines auf einer Anzeigeeinheit des Computers dargestellten Kurvendiagrammformulars anwählt, wodurch ein Eingabefenster aktiviert wird, in welches der Benutzer die patientenbezogenen Daten einträgt, wodurch der Computer innerhalb des Kurvendiagramms eine korrespondierende Markierung setzt.

## Beschreibung

Die Erfindung betrifft ein Verfahren zur Erstellung virtueller Kurvendiagramme, welche patientenbezogene Daten aufweisen, durch einen Benutzer eines Computers.

Seit langer Zeit werden in Krankenhäusern oder auch Pflegeeinrichtungen umfangreiche Dokumentationen über den gesundheitlichen Zustand der Patienten vorgenommen. Dabei werden insbesondere händische Eintragungen in sogenannten Patiententaschen vorgenommen, wobei der Arzt oder auch das Pflegepersonal medizinische Daten des Patienten wie beispielsweise Körpertemperatur, Puls, Blutdruck, Harnverhalten, Stuhlverhalten, die Einnahme von Medikamenten und so weiter in spezielle Formulare einträgt. Dabei werden diese Daten sowohl in Tabellenform als auch in der Form von Kurvendiagrammen dargestellt. Ein Formular kann dabei beispielsweise ein Kurvendiagramm enthalten, welches auf der x-Achse die Zeit und auf der y-Achse den zu dokumentierenden Messwert aufweist. Dieses Kurvendiagramm-Formular wird dabei so benutzt, dass der Benutzer zur üblichen Tageszeit, beispielsweise vormittags und/oder nachmittags den gewünschten Parameter des Patienten misst. Dieser Parameter kann beispielsweise die Körpertemperatur sein. Misst der Benutzer beispielsweise eine Körpertemperatur von 37 °C, trägt er diese Temperatur in das Kurvendiagramm bei dem jeweiligen Datum und der betreffenden Tageszeit und/oder Uhrzeit ein. Indem die zu unterschiedlichen Zeiten in das Kurvendiagramm eingetragenen Messwerte miteinander verbunden werden, entsteht eine Messkurve, welche den Temperaturverlauf des Patienten wiedergibt. Anhand dieses Kurvendiagramms kann das Pflegepersonal oder auch der behandelnde Arzt schnell und anschaulich erkennen, ob die Temperatur des Patienten über einem kritischen Wert liegt und wie der bisherige Temperaturverlauf war.

Die zwischenzeitlich rasant ansteigenden Anforderungen an das Pflegepersonal und auch die immer mehr in den Alltag eingeführte Verwendung von Computern erfordern auch ein Umdenken im Bereich der Patientenversorgung, um den immer höheren Anforderungen an präzise Patientendokumentation, Zeit- und Kosteneffizienz gerecht zu werden. In diesem Kontext ist auch die vorliegende Erfindung zu sehen. Insbesondere soll eine Möglichkeit geschaffen werden, die Patientendokumentation ohne eine fehlerbehaftete langwierige Umgewöhnungsphase von dem händischen auf ein computerbasiertes Verfahren umzustellen.

In diesem Sinne ist es Aufgabe der vorliegenden Erfindung, ein Verfahren zur Erstellung virtueller Kurvendiagramme, welche patientenbezogene Daten aufweisen, durch einen Benutzer eines Computers, zu schaffen, welches intuitiv von den Benutzern verwendet werden kann und insbesondere eine fehlerträchtige Eingewöhnungszeit bei der Umstellung von der händischen Dokumentation auf die virtuelle Dokumentation vermeidet.

Zu diesem Zweck wird ein vorgenanntes Verfahren zur Erstellung virtueller Kurvendiagramme vorgeschlagen, bei welchem der Benutzer mittels einer Eingabeeinheit des Computers einen Bereich eines auf einer Anzeigeeinheit des Computers dargestellten Kurvendiagrammformulars anwählt, wodurch ein Eingabefenster aktiviert wird, in welches der Benutzer die patientenbezogenen Daten einträgt, wodurch der Computer innerhalb des Kurvendiagramms eine korrespondierende Markierung setzt.

Durch das erfindungsgemäße Verfahren wird dem Benutzer auf einfache Art und Weise ermöglicht, die bisher händisch durchgeführte Patientendokumentation nunmehr in Verbindung mit modernen Computersystemen zu verwenden, so dass der Arzt oder auch das Pflegepersonal die Dokumentation relevanter Patienteninformationen, beispielsweise mit Hilfe eins Laptops unmittelbar im Zimmer des Patienten durchführen kann. Dadurch, dass das Verfahren dabei die bisher händisch getätigten Schritte mit technischen Mitteln nachahmt, entfällt ein langwieriger, fehleranfälliger Umstellungsprozess. Durch die Erstellung von Kurvendiagramm in virtueller Form auf einem Computer kann darüber hinaus auch die Zentralisation der Informationen sichergestellt werden, so dass stets von unterschiedlichen Personen an unterschiedlichen Orten auf die benötigten aktuellen Patienteninformationen zugegriffen werden kann. Dies erhöht die Qualität der medizinischen und pflegerischen Bemühungen, weil stets alle mit dem jeweiligen Patienten befassten Benutzer auf dem aktuellen Stand der Informationen sind.

Medizinische Werte werden durch die Eingabe des Benutzers dokumentiert und durch das erfindungsgemäße Verfahren als Kurvendiagramm gestaltet.

Diese Informationen sind maßgeblich für die Befundung durch den Arzt bei der Visite. Sie sind insbesondere entscheidend für die Anordnung der Medikation. Aus diesem Grunde wurde die Erfassung der Medikation in unmittelbare Nähe zu dem Kurvendiagramm gesetzt. Diese gewählte Struktur des erfindungsgemäßen Verfahrens erleichtert den Entscheidungsträgern im klinischen Alltag die Übersicht über relevante Daten.

Grundsätzlich wird von zwei unterschiedlichen Verantwortungsbereichen ausgegangen werden. Zum einen von der Anordnungsverantwortung des Arztes mit der Vorgabe, dass nachvollziehbar zu dokumentieren ist, wer hat, was, wann, wem, warum an Medikation verordnet. Zum anderen ist nachvollziehbar darzustellen, welche Person in die Durchführungsverantwortung eintritt, etwa durch das Richten der Medikation, der Aushändigung der Medikation an den Patienten oder die Verabreichung der Medikation an den Patienten. Das erfindungsgemäße Verfahren trägt diesen Anforderungen Rechnung, indem der Arzt zuvor eine passwortgeschützte Anmeldung durchführen muss und die einzelne Anordnung in Art, Dosis und Zeitraum durch Handzeichen legitimiert.

Gleiches gilt für das Pflegepersonal, welches ebenfalls passwortgeschützt die Anordnungen des Arztes nach Umsetzung mit Handzeichen dokumentiert.

Im Einzelnen erstellt der Benutzer ein virtuelles Kurvendiagramm, welches patientenbezogene Daten, insbesondere über den Gesundheitszustand, enthält, indem er mittels einer Eingabeeinheit des Computers einen Bereich eines auf einer Anzeigeeinheit des Computers dargestellten Kurvendiagrammformulars anwählt. Insbesondere kann die Eingabeeinheit dabei eine Computermaus sein, mittels der der Benutzer auf einen bestimmten Bereich des dargestellten Formulars klickt. Alternativ zu einer Computermaus oder auch einer Tastatur kann besonders einfach auch ein berührungssensitiver Bildschirm (Touchscreen) verwendet werden, so dass sich besonders vorteilhaft für den Benutzer das Gefühl ergibt, mit dem Finger in ein händisch ausgefülltes Kurvendiagrammformular zu zeigen. Durch das vorgenannte Anwählen eines Bereiches auf der Anzeigeeinheit wird ein Eingabefenster aktiviert, in welches der Benutzer sodann die patientenbezogenen Daten einträgt. Diese Daten können insbesondere die Körpertemperatur des Patienten, der Puls oder auch der Blutdruck sein. Die jeweilige Eingabe erfolgt dabei sinngemäß mittels eines Verweises auf das aktuelle Tagesdatum und die Tageszeit oder insbesondere Uhrzeit.

Durch den Eintrag wird innerhalb des Kurvendiagramms eine korrespondierende Markierung gesetzt, so dass die Eingabe des Benutzers zu der Erstellung eines Kurvendiagramms in der Form führt, welche er bereits von dem in Papierform vorliegenden Formular kennt. Sofern nun zu aufeinanderfolgenden Zeitpunkten solche Markierungen gesetzt werden, entsteht ein Kurvendiagramm, welches auf der x-Achse die Zeit und auf der y-Achse den jeweiligen Messwert aufweist. Somit kann der Arzt oder auch das Pflegepersonal auf einen Blick erkennen, ob die Körpertemperatur oder auch ein anderer Gesundheitsparameter des Patienten im normalen Bereich liegt oder nicht, um gegebenenfalls therapeutische Maßnahmen zu ergreifen.

Eine Ausführungsform der Erfindung sieht dabei vor, dass das Eingabefenster eine Darstellung, insbesondere eine vergrößerte Darstellung, des Kurvendiagrammformulars enthält, wobei der Benutzer mittels der Eingabeeinheit unmittelbar eine Markierung innerhalb des Kurvendiagramms setzt. Dabei gibt diejenige Position innerhalb des Kurvendiagramms, auf welche der Benutzer mittels der Computermaus oder auch des Touchscreens klickt, denjenigen Ort an, an welchem auch die Markierung gesetzt wird. Dabei hat der Benutzer darauf zu achten, dass er die für die Markierung gewünschte Stelle, welche der aktuellen Zeit und dem gemessenen Messwert entspricht, an die richtige Stelle setzt. Besonders vorteilhaft ist dabei vorgesehen, dass sich bei der Anwahl des Bereichs des Kurvendiagrammformulars durch den Benutzer eine vergrößerte Darstellung des angewählten Bereiches ausbildet, ähnlich wie bei einer sogenannten Lupendarstellung, so dass der Benutzer leicht den gewünschten Markierungsbereich innerhalb des Zeit-Messwert-Diagrammes erkennt und seine Markierung setzen kann.

Eine alternative Ausgestaltung der Erfindung sieht vor, dass das Eingabefenster Eingabebereiche enthält, in welche der Benutzer mittels der Eingabeeinheit, insbesondere mittels einer Tastatur, die patientenbezogenen Daten, insbesondere Zahlenwerte, einträgt. Gemäß dieser Ausgestaltung erzeugt die Anwahl eines Bereiches innerhalb des Kurvendiagrammformulars und die Aktivierung eines Eingabefensters nicht unmittelbar eine Markierung innerhalb des Kurvendiagramms, sondern der Benutzer wird stattdessen aufgefordert, in dafür vorgesehene Eingabebereiche des Eingabefensters Angaben über beispielsweise Datum, Tageszeit, Uhrzeit, Temperaturmesswert und so weiter einzutragen. Sobald der Benutzer die entsprechenden Eingaben getätigt hat, bestätigt er seine Eingabe beispielsweise mittels eines Mausklicks oder auch der Return-Taste auf der Tastatur der Eingabeeinheit, so dass anschließend eine den Eingabeparametern entsprechende Markierung innerhalb des Kurvendiagramms erscheint.

Weiterhin ist vorgesehen, dass der Benutzer in das Eingabefenster zusätzlich eine ihn identifizierende Information einträgt. Diese ihn identifizierende Information kann dabei beispielsweise sein Familienname oder auch ein Namenkürzel sein. Dadurch wird sichergestellt, dass stets erkennbar ist, welcher Benutzer die jeweilige Markierung innerhalb des Kurvendiagramms gesetzt hat. Dadurch werden entsprechende Rückfragen bei der zuständigen Person vereinfacht.

Die Erfindung sieht weiterhin vor, dass in Bezug auf die x-Achse des Kurvendiagramms ein Zeitverlauf, insbesondere eine Angabe über Datum, Tageszeit und/oder Uhrzeit, eingetragen wird, und dass in Bezug auf mindestens eine y-Achse des Kurvendiagramms Informationen über eine Körpertemperatur, den Puls oder einen Blutdruck des Patienten eingetragen werden. Dabei kann das Kurvendiagramm gleichzeitig mehrere y-Achsen aufweisen, so dass der Benutzer in einem gemeinsamen Diagramm alle relevanten Informationen vorfindet. Besonders vorteilhaft können dabei die Normwerte für Körpertemperatur, Puls und Blutdruck auf denselben y-Achsenabstand gelegt werden, so dass eine Abweichung vom Normwert auf einen Blick erkannt werden kann. Der Normwert kann dabei zusätzlich als Schwellenwert optisch besonders herausgestellt werden, beispielsweise in Form einer roten Linie, welche parallel zu der x-Achse verläuft.

Vorteilhaft wird in Bezug auf die Information über die Körpertemperatur zusätzlich eine Information darüber eingetragen, an welchem Körpermesspunkt diese Temperatur gemessen wurde. Der Ort des Körpermesspunktes ist dabei von besonderer Wichtigkeit, da sich die gemessene Temperatur je nach dem Messort durchaus stark unterscheiden kann. So ergeben sich unterschiedliche Messpunkte beispielsweise bei der Axillar-, Rektal-, Sublingual-Messung oder auch der Messung im Ohr.

Dabei empfiehlt es sich insbesondere, dass der Benutzer die patientenbezogenen Daten abhängig von der Art der Information bei der Eintragung in das Kurvendiagramm einer charakteristischen Farbe oder Form zuordnet. So können beispielsweise Messungen im Ohr eine punktförmige Markierung aufweisen, während Axillar-Messungen mittels einer quadratischen Markierung gekennzeichnet werden. Ebenso lassen sich auch unterschiedliche Markierungen für unterschiedliche Körperparameter wie beispielsweise Temperatur, Puls oder Blutdruck, mit unterschiedlichen Formen oder auch Farben kennzeichnen.

Obwohl die vorgenannte Erfindung bisher anhand eines Verfahrens verdeutlicht wurde, soll in die Erfindung insbesondere auch ein Computerprogrammprodukt einbezogen sein, mittels welchem sich das Verfahren zur Erstellung virtueller Kurvendiagramme ausführen lässt. Ebenso ist in die Erfindung ein Computer mit einem Computerprogramm zur Ausführung des Verfahrens zur Erstellung virtueller Kurvendiagramme miteinbezogen.

Im Folgenden wird die Erfindung anhand eines Ausführungsbeispiels näher erläutert:
Auf einer Krankenstation verfügen die Ärzte und/oder die Pflegekräfte über einen Computer, insbesondere einen Laptop, auf welchem ein Computerprogramm zur Ausführung des erfindungsgemäßen Verfahrens installiert ist. Dabei kann beispielsweise jeder Arzt einen eigenen Computer besitzen, während sich die Pflegekräfte beispielsweise einen Computer gemeinsam teilen. Über Schnittstellen können die Computer synchronisiert werden, so dass jeder Benutzer aktuell die gleichen Informationen auf seinem Computer vorfindet.

Geht eine Pflegekraft (im Folgenden Benutzer genannt) mit einem Laptop, mittels welchem das erfindungsgemäße Verfahren ausführbar ist, in das Zimmer eines Patienten, führt sie die regelmäßigen Untersuchungen durch. Diese Untersuchungen können beispielsweise eine Messung der Körpertemperatur, des Pulses, des Blutdrucks oder auch die Aufnahme von sprachlichen Informationen umfassen. Nach der Untersuchung öffnet der Benutzer auf seinem Computer ein leeres oder auch bereits in Benutzung genommenes Kurvendiagrammformular. Sofern dies noch nicht erfolgt ist, trägt der Benutzer allgemeine Informationen über den Patienten, beispielsweise dessen Namen und Alter ein, und ordnet somit das neue, noch unbenutzte Kurvendiagrammformular dem jeweiligen Patienten zu. Um nun einen neuen Messwert in das Kurvendiagrammformular einzubeziehen, wählt der Benutzer mittels der Eingabeeinheit des Computers, welche eine Computermaus oder auch ein Touchscreen sein kann, einen Bereich des auf der Anzeigeeinheit, beispielsweise des Monitors, des Computers dargestellten Kurvendiagrammformulars an. Diese Anwahl erfolgt dabei vorteilhaft durch ein Klicken in das jeweilige Kurvendiagrammformular. Dadurch wird ein Eingabefenster aktiviert, in welches der Benutzer den Messwert einträgt. Durch diesen Eintrag wiederum setzt der Computer innerhalb des Kurvendiagramms eine korrespondierende Markierung.

Im Sinne der "Aktivierung" des Eingabefensters kommen zwei unterschiedliche Verfahrensschritte in Frage. Zum einen kann durch das Anwählen des Kurvendiagrammformulars mittels eines Mausklicks eine vergrößerte Darstellung des aktivierten Bereiches des Kurvendiagrammformulars geschaffen werden, wobei der Benutzer mittels der Eingabeeinheit unmittelbar eine Markierung innerhalb des Kurvendiagramms setzt. Dies funktioniert für den Benutzer genauso, als wenn er mittels eines Stiftes in ein als Papierversion vorliegendes Kurvendiagrammformular schreiben würde. Somit wird die Markierung innerhalb des Kurvendiagramms unmittelbar durch die Eingabe des Benutzers erzeugt. Alternativ kann durch die Anwahl des Kurvendiagrammformulars aber auch nur ein Eingabefenster öffnen, welches mehrere Eingabebereiche enthält, die der Benutzer zunächst auszufüllen hat. Diese Eingabebereiche können Eingabefelder für die Messwerte, das Datum, den Namen des Benutzers oder ähnliches sein. Erst durch die Bestätigung der Eingabe wird anschließend die Markierung innerhalb des Kurvendiagramms erzeugt.

Gleichzeitig mit der Eingabe der Daten über den gesundheitlichen Zustand kann der Benutzer eine Information darüber eintragen, an welchem Körpermesspunkt die entsprechenden Daten gemessen wurden. Dies betrifft insbesondere die Messung der Körpertemperatur, da hier unterschiedliche Körpermesspunkte - wie beispielsweise bei der Axillar-, Rektal-, Sublingual-Messung oder auch der Messung im Ohr - in Frage kommen. Damit der Benutzer anschließend direkt optisch erkennen kann, an welchem Messpunkt die jeweiligen Messungen durchgeführt wurden, können die unterschiedlichen Messpunkte optisch durch unterschiedliche Farben oder Formen markiert sein. Dabei kann beispielsweise eine Axillar-Messung mittels einer runden Markierung angezeigt werden, während eine Rektal-Messung durch ein Quadrat angezeigt wird. Zusätzlich können sich auch unterschiedliche Messparameter, beispielsweise Temperatur, Puls und so weiter, durch ihre Farbigkeit unterscheiden. Vorteilhaft ist ebenfalls, wenn innerhalb des Kurvendiagramms Schwellwerte angeordnet sind, welche optisch unmittelbar das Über- bzw. Unterschreiten eines Standardmesswertes anzeigen.

Durch das so erstellte Kurvendiagramm erhält der Benutzer unmittelbar einen optischen Eindruck von dem Gesundheitszustand des Patienten.

Der Dokumentation von Änderungen trägt das erfindungsgemäße Verfahren in besonderer Weise Rechnung, da diesen im klinischen Alltag besondere Bedeutung zukommt und sie daher besonderer Kennzeichnung bedürfen. Als Änderungen werden nachträgliche Veränderungen von Einträgen bezeichnet, die nicht innerhalb der gleichen Session erfolgen, sondern etwa nach einem Schließen des Formularbogens.

Jegliche Änderung von Werteinträgen im erfindungsgemäßen Verfahren wird optisch dargestellt, als "Historie Wert" gespeichert und steht in der kompletten digitalen Patientenakte des erfindungsgemäßen Verfahrens zur Verfügung.

Die optische Darstellung erfolgt:
Bei Änderungen von "Texteinträgen" durch eine auffällige rote Schrift.
Bei Änderungen von "Kurvendarstellungen" durch eine rote Einrahmung.
Bei Änderungen von "Auswahlfeldern (z.B. Radiobutton)" durch ein rotes "H".

Änderungen müssen in jedem Fall mit einer "schriftlichen" Begründung versehen werden, welche zusammen mit den abgeänderten Werten, in der Historie des erfindungsgemäßen Verfahrens gespeichert wird.

Durch Synchronisation mehrerer Computer lässt sich ebenfalls erreichen, dass mehrere Ärzte oder auch Pflegekräfte, welche mit dem Patienten befasst sind, immer auf dem gleichen aktuellen Stand sind.

## Patentansprüche

1. Verfahren zur Erstellung virtueller Kurvendiagramme, welche patientenbezogene Daten aufweisen, durch einen Benutzer eines Computers,
**dadurch gekennzeichnet,**
**dass** der Benutzer mittels einer Eingabeeinheit des Computers einen Bereich eines auf einer Anzeigeeinheit des Computers dargestellten Kurvendiagrammformulars anwählt, wodurch ein Eingabefenster aktiviert wird, in welches der Benutzer die patientenbezogenen Daten einträgt, wodurch der Computer innerhalb des Kurvendiagramms eine korrespondierende Markierung setzt.

2. Verfahren nach Anspruch 1, **dadurch gekennzeichnet, dass** das Eingabefenster eine, insbesondere vergrößerte, Darstellung des aktivierten Bereiches des Kurvendiagrammformulars enthält, wobei der Benutzer mittels der Eingabeeinheit unmittelbar eine Markierung innerhalb des Kurvendiagramms setzt.

3. Verfahren nach Anspruch 1, **dadurch gekennzeichnet, dass** das Eingabefenster Eingabebereiche enthält, in welche der Benutzer mittels der Eingabeeinheit, insbesondere mittels einer Tastatur, die patientenbezogene Daten, insbesondere Zahlenwerte, einträgt.

4. Verfahren nach einem der vorhergehenden Ansprüche, **dadurch gekennzeichnet, dass** der Benutzer in das Eingabefenster zusätzlich eine ihn identifizierende Information einträgt.

5. Verfahren nach einem der vorhergehenden Ansprüche, **dadurch gekennzeichnet, dass** in Bezug auf die x-Achse des Kurvendiagramms ein Zeitverlauf, insbesondere eine Angabe über Datum, Tageszeit und/oder Uhrzeit, eingetragen wird, und dass in Bezug auf mindestens eine y-Achse des Kurvendiagramms Informationen über eine Körpertemperatur, den Puls oder einen Blutdruck des Patienten eingetragen werden.

6. Verfahren nach Anspruch 5, **dadurch gekennzeichnet, dass** in Bezug auf die Information über die Körpertemperatur zusätzlich eine Information darüber eingetragen wird, an welchem Körpermesspunkt diese Temperatur gemessen wurde.

7. Verfahren nach einem der vorhergehenden Ansprüche, **dadurch gekennzeichnet, dass** der Benutzer die patientenbezogenen Daten abhängig von der Art der Information bei der Eintragung in das Kurvendiagramm einer charakteristischen Farbe oder Form zuordnet.

8. Computerprogrammprodukt, mittels welchem sich das Verfahren zur Erstellung virtueller Kurvendiagramme gemäß einem der Ansprüche 1 bis 7 ausführen lässt.

9. Computer mit einem Computerprogramm zur Ausführung des Verfahrens zur Erstellung virtueller Kurvendiagramme gemäß einem der Ansprüche 1 bis 7.
